# EUROPEAN PATENT APPLICATION

(11) **EP 2 703 000 A1**
(43) Date of publication of application: **05.03.2014**
(21) Application number: 11864172.9
(22) Date of filing: 26.05.2011
(51) Int. Cl.: A61K 31/715, A61K 36/8968, A61P 31/04

(54) **APPLICATION OF DWARF LILYTURF TUBER POLYSACCHARIDE EXTRACT IN PREPARATION OF DIETARY SUPPLEMENT, HEALTH FOOD OR MEDICINE WITH THE FUNCTION OF WEIGHT LOSS**

(30) Priority: 25.04.2011 CN 201110103158
(71) Applicant: Shanghai Zhangjiang Engineering Research Center of Modern Preparation Technology of Traditional Chinese Medicine, Shanghai 201203 (CN)
(72) Inventor: FENG, Yi, Shanghai 201203 (CN); XU, Desheng, Shanghai 201203 (CN); RUAN, Kefeng, Shanghai 201203 (CN); WANG, Shuo, Shanghai 201203 (CN); LIN, Xiao, Shanghai 201203 (CN); LIANG, Shuang, Shanghai 201203 (CN); WANG, Yuan, Shanghai 201203 (CN)
(74) Representative: Brown, David Leslie
(86) International application number: PCT/CN2011/074709
(87) International publication number: WO 2012/145946

(57) **Abstract**

A usage of the extracts from polysaccharide of *Ophiopogon japonicus* in the preparation of food additives, health products or pharmaceutical products.

## Description

### Technical Field

The invention relates to a new usage of the extracts from polysaccharide of *O. japonicus,* and in particular to the usage of the extracts from polysaccharide of *O. japonicus* in the preparation of food additives, health products or pharmaceutical products with weight reducing effects.

### Background Art

Obesity is a disease of nutritional dysbolism caused by interplaying factors of heritage and the environment, and is a major inducer of various chronic diseases. Frequently accompanied by insulin resistance and hyperinsulinemia, it can induce cardiovascular disease related abnormality in various metabolic functions, and may increase incidence and mortality rate of type II diabetes, coronary diseases, stroke, congestive heart failure, hypertension, dyslipidemia, obstructive sleep apnea, and certain cancers (such as ovary cancer, thymic carcinoma, and colon carcinoma).

Yin-nourishing Chinese herbs have the effects of producing fluids, moisturizing dryness, and nourishing Yin and fluids. They mostly treat the Yin deficiency syndrome, have low toxicity and mild effects, with their main functions being nourishing, and therefore are significant for strengthening human immunity and for disease prevention. As the pharmacology of the polysaccharide gets better understood, more research is being carried out on it, and it is shown that the Yin-nourishing Chinese herbs have a high content of polysaccharide, with their physiological activities being exhibited by the effects of: immune regulation, anti-tumour, hypoglycemic, lipid-lowering, anti-oxidation, anti-aging, hepatoprotective, antiviral, anti-fatigue, anti-anoxia, anti-fibrotic, and anti-ulcer.

No literature is found in the prior art on the usage of the extracts from the polysaccharide of *O. japonicus* with a range of peak molecular weight in 3000~6500 with weight reducing effects.

### Summary of the Invention

The technical problem which the present invention solves is to provide a usage of the extracts from polysaccharide of *O. japonicus* in the preparation of food additives, health products or pharmaceutical products with weight reducing effects.

The extracts from polysaccharide of *O. japonicus* of the present invention has a range of peak molecular weight in 3000~6500, and is obtained by separation from the total polysaccharides of *O. japonicus.* The method of separation has the following steps:

### 1) Extraction of total polysaccharides of O. japonicus:

Take multiple dry tuberous roots of *O. japonicus,* reflux them three times with 70 %-95 % ethanol 5-6 times of their weight, discharge the roots, extract decoction 2-3 times therefrom, concentrate the aqueous decoction under reduced pressure to a concentration of 0.5-1 g/ml, add 75%-95% ethanol until the ethanol concentration reaches 60%-80%, stand the decoction overnight, discharge the supernatant, carry out ethanol precipitation twice, dry the decoction to obtain a powder of total polysaccharides of *O. japonicus,* which is in pale yellow color;

### 2) Purification of the extracts from the polysaccharide of O. japonicus:

Take the total polysaccharides of *O. japonicus,* add in water for dissolution, discharge the sediment by centrifugation; into the supernatant add 1-2 times its volume 70%-95% ethanol to form sedimentation, stand the supernatant overnight, and discharge the sediment; into the supernatant add once again 1-2 times its volume 70%-95% ethanol to form sedimentation again, stand the supernatant overnight, and collect the sediment, which is an extract of the polysaccharide of *O. japonicus* (with a range of molecular weight in 5000~6000); in the remaining supernatant add in 4-5 times of its volume 70%-95% ethanol to form sedimentation, stand the supernatant overnight, and collect the sediment, which is an extract of the polysaccharide of *O. japonicus* (with peak molecular weight in 3000~5000).

The present invention employs the model of belly-fat rats to investigate the experiment of the inhibition of the extracts from the polysaccharide of *O. japonicus* on weight gain of the fat rats, to evaluate the weight reducing effects of the extracts from the polysaccharide of *O. japonicus.* The experiment carried out on the belly-fat rats shows that the extracts from the polysaccharide of *O. japonicus* does not affect the food intake quantity of the rats, and that after being administered a high dose of the extracts from the polysaccharide of *O. japonicus* for one week, the weight gain of the rats is substantially mitigated, and the effects of the weight gain inhibition last for 14 weeks till the conclusion of the experiment. Compared with those in the model group, the belly-fat rats administered a high dose of the extracts from the polysaccharide of *O. japonicus* show a substantial decrease in the rate of their weight gain. The outcome of the experiments shows that: the extracts from the polysaccharide of *O. japonicus* of the present invention possesses a prominent weight reducing effect, and is applicable in the preparation of food additives, health products or pharmaceutical products with weight reducing effect.

As food additive or health product, the extracts from the polysaccharide of *O. japonicus* of the present invention can be added into drinking water, foodstuff, beverage, alcohol, condiments, milk products, edible oil, flour food, etc., to exhibit its weight reducing effect.

As health product or pharmaceutical product, the extracts from the polysaccharide of *O. japonicus* of the present invention can be fabricated in various oral dosage forms, including tablets, capsules, granules or oral solution. The oral dosage preferably is a composite of the extracts from the polysaccharide of *O. japonicus* 5%-60% by weight with pharmaceutical excipients or additives 95%-40% by weight.

### Description of the Drawing

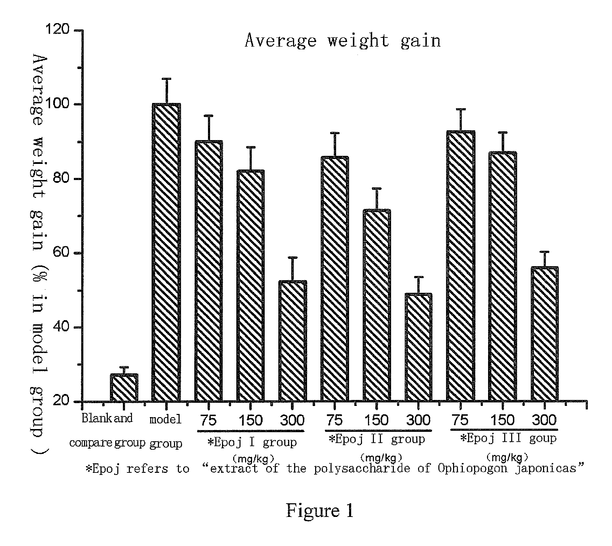

Figure 1 is the schematic diagram of the mean weight gain of belly-fat rats of all the groups in the Experiment 1 of the present invention.

### Embodiments

The present invention will be elucidated in more details with the following embodiments:
Embodiment 1 preparation of the extracts from the polysaccharide of *O. japonicus* (with peak molecular weight 5000~6500)
1) Extraction of total polysaccharides of *O. japonicus:*
   Take 1kg of tuberous roots of *O. japonicus,* squash and reflux them three times with 80 % ethanol 3 times of their weight, one hour each time, extracts decoction 2-3 times therefrom, 30 minutes each time. Concentrate the aqueous decoction under reduced pressure to a concentration of 1 g/ml, add 95% ethanol until the ethanol concentration reaches 60%-80%, stand the decoction overnight, discharge the supernatant, carry out ethanol precipitation twice, freeze-dry the decoction to obtain a powder of total polysaccharides of *O. japonicus,* which is in pale yellow color;
2) Purification of the extracts from the polysaccharide of *O. japonicus* (with peak molecular weight 5000~6500):
   Take the total polysaccharides of *O. japonicus,* add in water for dissolution, discharge the sediment by centrifugation. Into the supernatant add 1-2 times its volume 70%-95% ethanol to form sedimentation, stand the supernatant overnight, and discharge the sediment. Into the supernatant add once again 1-2 times its volume 70%-95% ethanol to form sedimentation again, stand the supernatant overnight, and collect the sediment, which is an extracts from the polysaccharide of *O. japonicus* (with peak molecular weight in 5000~6500).

Embodiment 2 prepareation of the extracts from the polysaccharide of *O. japonicus* (with peak molecular weight 4000~5000)
1) Extraction of total polysaccharides of *O. japonicus:*
   Take dry tuberous roots of *O. japonicus,* reflux them three times with 70%-95% ethanol 5-6 times of their weight, discharge the pulp, extracts decoction 2-3 times therefrom, concentrate the aqueous decoction under reduced pressure to a concentration of 0.5-1 g/ml, add 75%-95 % ethanol until the ethanol concentration reaches 60%-80%, stand the decoction overnight, discharge the supernatant, carry out ethanol precipitation twice, dry the decoction to obtain a powder of total polysaccharides of *O. japonicus,* which is in pale yellow color;
2) Purification of the extracts from the polysaccharide of *O. japonicus* (with peak molecular weight 4000~5000):
   Take the total polysaccharides of *O. japonicus,* add in water for dissolution, discharge the sediment by centrifugation. Into the supernatant add 1-2 times its volume 70%-95% ethanol to form sedimentation, stand the supernatant overnight, and discharge the sediment. Into the supernatant add once again 1-2 times its volume 70%-95% ethanol to form sedimentation again, stand the supernatant overnight, separate the sediment and collect the supernatant. Into the supernatant add once again 1-2 times its volume 70%-95% ethanol to form sedimentation again, stand the supernatant overnight, and collect the sediment, which is an extracts from the polysaccharide of *O. japonicus* (with peak molecular weight in 4000~5000).

Embodiment 3 Preparation of the extract of the polysaccharide of *O. japonicus* (with peak molecular weight 3000~4000)
1) Extraction of total polysaccharides of *O. japonicus:*
   Take dry tuberous roots of *O. japonicus,* reflux them three times with 70%-95% ethanol 5-6 times of their weight, discharge the roots, extract decoction 2-3 times therefrom, concentrate the aqueous decoction under reduced pressure to a concentration of 0.5-1 g/ml, add 75%-95% ethanol until the ethanol concentration reaches 60%-80%, stand the decoction overnight, discharge the supernatant, carry out ethanol precipitation twice, dry the decoction to obtain a powder of total polysaccharides of *O. japonicus,* which is in pale yellow color;
2) Purification of the extracts from the polysaccharide of *O. japonicus* (with peak molecular weight 3000~4000):
   Take the total polysaccharides of *O. japonicus,* add in water for dissolution, discharge the sediment by centrifugation. Into the supernatant add 1-2 times its volume 70%-95% ethanol to form sedimentation, stand the supernatant overnight, and discharge the sediment. Into the supernatant add once again 1-2 times its volume 70%-95% ethanol to form sedimentation again, stand the supernatant overnight, separate the sediment and collect the supernatant. Into the supernatant add once again 3-4 times its volume 70%-95% ethanol to form sedimentation again, stand the supernatant overnight, and collect the sediment, which is an extract of the polysaccharide of *O. japonicus* (with peak molecular weight in 3000~4000).

Embodiment 4 Fabrication of tablets with contents of the extract of the polysaccharide of *O. japonicus*
Formula (1000 tablets):
Extract of the polysaccharide of *O. japonicus* 100 g (prepared according to in Embodiment 1)

| | |
|---|---|
| Microcrystalline Cellulose | 37.45 g |
| Precipitate Silica | 10.5 g |
| Stearic Acid | 17.5 g |
| Lactose | 10.5 g |
| Magnesium Stearate | 1.05 g |

Mix microcrystalline cellulose, precipitate silica, stearic acid, and lactose according to the formula with the extract of the polysaccharide of *O. japonicus* uniformly, and prepare into granules, add magnesium stearate and mix uniformly, compress into 1000 tablets (each tablet with 0.1g of the extracts from the polysaccharide of *O. japonicus).* Recommended adult dosage is 1-4 tablets/time, 2-3 times per day.
Embodiment 5 Fabrication of capsules of the extracts from the polysaccharide of *O. japonicus*

| | |
|---|---|
| Formula (1000 capsules): | |
| Extract of the polysaccharide of *O. japonicus* 100 g (fabricated as in Embodiment 1) | |
| Microcrystalline Cellulose | 50 g |
| Lactose | 30 g |

Mix microcrystalline cellulose, and lactose according to the formula with the extract of the polysaccharide of *O. japonicus* uniformly, and prepare into granules, encapsulate into 1000 capsules (each capsule with 0.1g of the extract of the polysaccharide of *O. japonicus).* Recommended adult dosage is 1-4 capsules/time, 2-3 times per day.
Embodiment 6 Fabrication of granules of the extracts from the polysaccharide of *O. japonicus*

| | |
|---|---|
| Formula (1000 bags): | |
| Extract of the polysaccharide of *O. japonicus* 100 g (fabricated as in Embodiment 1) | |
| Microcrystalline Cellulose | 38.5 g |
| Precipitate Silica | 10.5 g |
| Stearic Acid | 17.5 g |
| Lactose | 10.5 g |

Mix microcrystalline cellulose, precipitate silica, stearic acid, and lactose according to the formula with the extracts from the polysaccharide of *O. japonicus* uniformly, and prepare into granules, package into 1000 bags (each bag with 0.1g of the extract of the polysaccharide of *O. japonicus*)*.* Recommended adult dosage is 1-4 bags/time, 2-3 times per day.
Embodiment 7 Fabrication of beverage with contents of the extracts from the polysaccharide of *O. japonicus*

| | |
|---|---|
| Formula (1000 bottles): | |
| Extracts from the polysaccharide of *O. japonicus* 100 g (prepared according to Embodiment 1) | |
| Beverage | 5000 mL |

Add the extracts from the polysaccharide of *O. japonicus* into the 5000 mL beverage, and package into 1000 bottles.
Embodiment 8 Fabrication of biscuits with contents of the extracts from the polysaccharide of *O. japonicus*

| | |
|---|---|
| Formula (100 boxes): | |
| Extracts from the polysaccharide of *O. japonicus* 100 g (fabricated as in Embodiment 1) | |
| Butter | 800 g |
| Eggs | 1000 g |
| Cake Flour | 1800 g |
| Milk Powder | 400 g |
| Baking Powder | 30 g |

According to the formula, put baking powder into warm water, stir until dissolved, add cake flour and stir, and then add the other ingredients (extracts from the polysaccharide of *O. japonicus,* butter, eggs, milk powder), stir until uniformly mixed, and knead into smooth dough. Slice the dough into thin pieces, and ferment them in a warm location (38°C) for 10 minutes, bake, and package into boxes.

The present invention will be further elucidated with the following experiment which shows the pharmacological activity of the extracts from the polysaccharide of *O. japonicus* and the related results.

### Experiment 1: Effects of the extracts from the polysaccharide of O. japonicus on the weight gain and food intake quantity of rats with Diet-induced Obesity (DIO).

### 1. Animals

Select 220 rats, 4-5 weeks old, C57, male. Feeding conditions: central air-conditioning for control of temperature and humidity, feeding in cages specially suited for rats, with 3 rats in each box. Temperature 22±1°C, relative humidity 40%-60%, controlled illumination with 12 hours light and 12 hours in dark, with animals feeding and drinking voluntarily.

### 2. Drugs and agents

Extracts from the polysaccharide of *O. japonicus* I (peak molecular weight 5000~6500, self-prepared according to Embodiment 1); extracts from the polysaccharide of *O. japonicus* II (peak molecular weight 4000~5000, self-prepared according to Embodiment 2); extracts from the polysaccharide of *O. japonicus* III (peak molecular weight 3000~4000, self-prepared according to Embodiment 3); common feedstuff (purchased from Shanghai Slac Laboratory Animal Company Limited); high fat forage (60% fat content, D 12492, purchased from Research Diet in the United States); sodium chloride injection (purchased from Shijiazhuang No. 4 Pharmaceutical Company Limited).

### 3. Method of Experiment

### 3.1 Model construction of the fat-belly rats

The C57 rats in the blank group are fed with common feedstuff, the other C57 rats are fed with high fat forage, and all on voluntary feeding and drinking. On the 8^{th} week of the feeding, the rats are grouped according to their weights, with 12 in a group with uniform weights among groups.

### 3.2 Grouping design of the experiment animals

**Table 1**

| Group | dosage | Medication volume | Medication duration | Number of animals |
|---|---|---|---|---|
| | (mg/kg) | (mL/kg/dose) | (day) | ♂ |
| blank and compare group | -- | 10 | 98 | 12 |
| Model group | -- | 10 | 98 | 12 |
| Epoj I* | 75 | 10 | 98 | 12 |
| Epoj I* | 150 | 10 | 98 | 12 |
| Epoj I* | 300 | 10 | 98 | 12 |
| Epoj II* | 75 | 10 | 98 | 12 |
| Epoj II* | 150 | 10 | 98 | 12 |
| Epoj II* | 300 | 10 | 98 | 12 |
| Epoj III* | 75 | 10 | 98 | 12 |
| Epoj III* | 150 | 10 | 98 | 12 |
| Epoj III* | 300 | 10 | 98 | 12 |

| | | | | |
|---|---|---|---|---|
| *Epoj refers to "extracts from the polysaccharide of Ophiopogon japonicas". | | | | |

### 3.3 experimental drug delivery

The blank and compare group and the model group are administered double distilled water, while the epoj (extracts from the polysaccharide of *O. japonicus*) groups are administered the extracts from the polysaccharide of *O. japonicus* with corresponding concentration, one time per diem, for 98 consecutive days.

### 3.4 Quantity of food intake

Quantity of food intake of the rats in each group is measured once each week, and recorded.

### 3.5 Values of weight gain

Weights of rats in each group are measured once per week, and the values of the weight gain are calculated.

### 3.6 Statistical method

All data are shown in mean±standard deviation (x̅ ± *s*), and statistical analysis is carried out with SPSS 11.10 statistical software. Comparing of means among groups is *t*-tested, and comparing of means of multiple samples adopts single factor analysis of variance.

### 4. Results

Quantity of food intake of the rats in each group is measured regularly during the drug administration, and the result is shown on Table 2. During the whole length of the duration of drug administration, the mean daily food intake quantity of the rats in the nine groups administered the extracts from the polysaccharide of *O. japonicus* does not show a marked difference as compared with that of the rats in the model group. However, the food intake quantity of the rats in the ten groups administered high fat forage shows a marked decrease as compared with that of the rats administered common feedstuff in the blank and compare group. At the same time, weights of the rats are measured regularly during the drug administration, and the result is shown on Table 3, showing that the weights of the rats in each group experience a continual increase. Compared with the blank and compare group, the weights of the rats in the model group show a significant increase. During the whole length of the drug administration, as compared with the model group, the rats administered 300 mg/kg of the extracts from the polysaccharide of *O. japonicus* I, II and III for one week show a significant slowdown of weight gain, and the effects of the weight gain inhibition last for 14 weeks till the conclusion of the experiment, with the belly-fat rats administered 300 mg/kg of the extract of the polysaccharide of *O. japonicus* I, II and III showing a decreasing rate of mean weight gain of 47.7%, 51.2% and 44.1% respectively (see Figure 1). At the same time, the rats administered 150 mg/kg of the extracts from the polysaccharide of *O. japonicus* I, II and III also show a decreasing trend of weight gain, with the decreasing rates of the mean weight gain being 17.9%, 28.6% and 13.0% respectively. The decreasing rates of the mean weight gain of the rats administered 75 mg/kg of the extracts from the polysaccharide of *O. japonicus* I, II and III are 9.9%, 14.3% and 7.3% respectively. The above results show that the extracts from the polysaccharide of *O. japonicus* (with peak molecular weight in the range of 3000~6500) purified from the total polysaccharides of *O. japonicus* inhibits weight gain of the belly-fat rats significantly, and shows a prominent effect in weigh reduction, and can be employed in the preparation of food additives, health products or pharmaceutical products with weight reducing effects.

The numbers "1-14" in the first line of Table 2 and three means the number of weeks from the time of drug delivery.

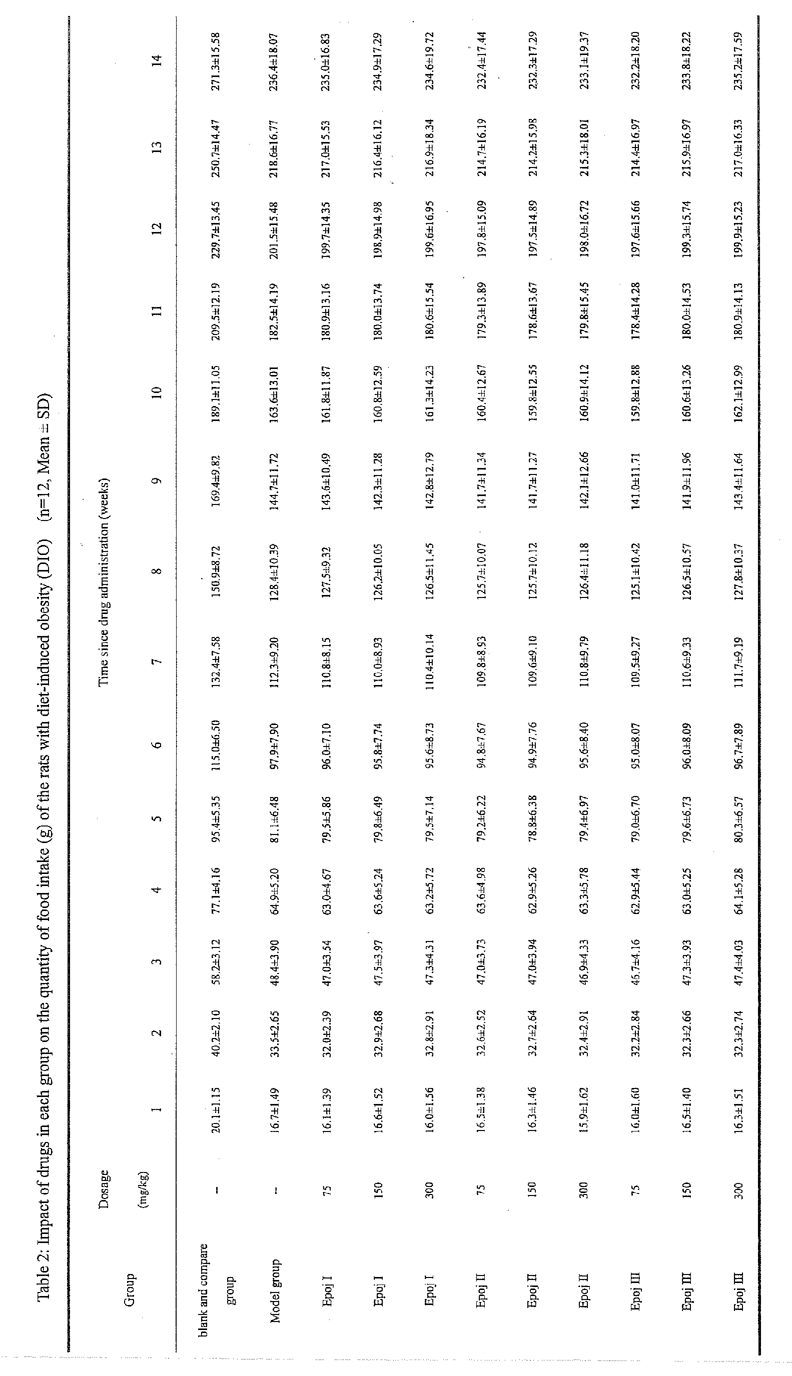

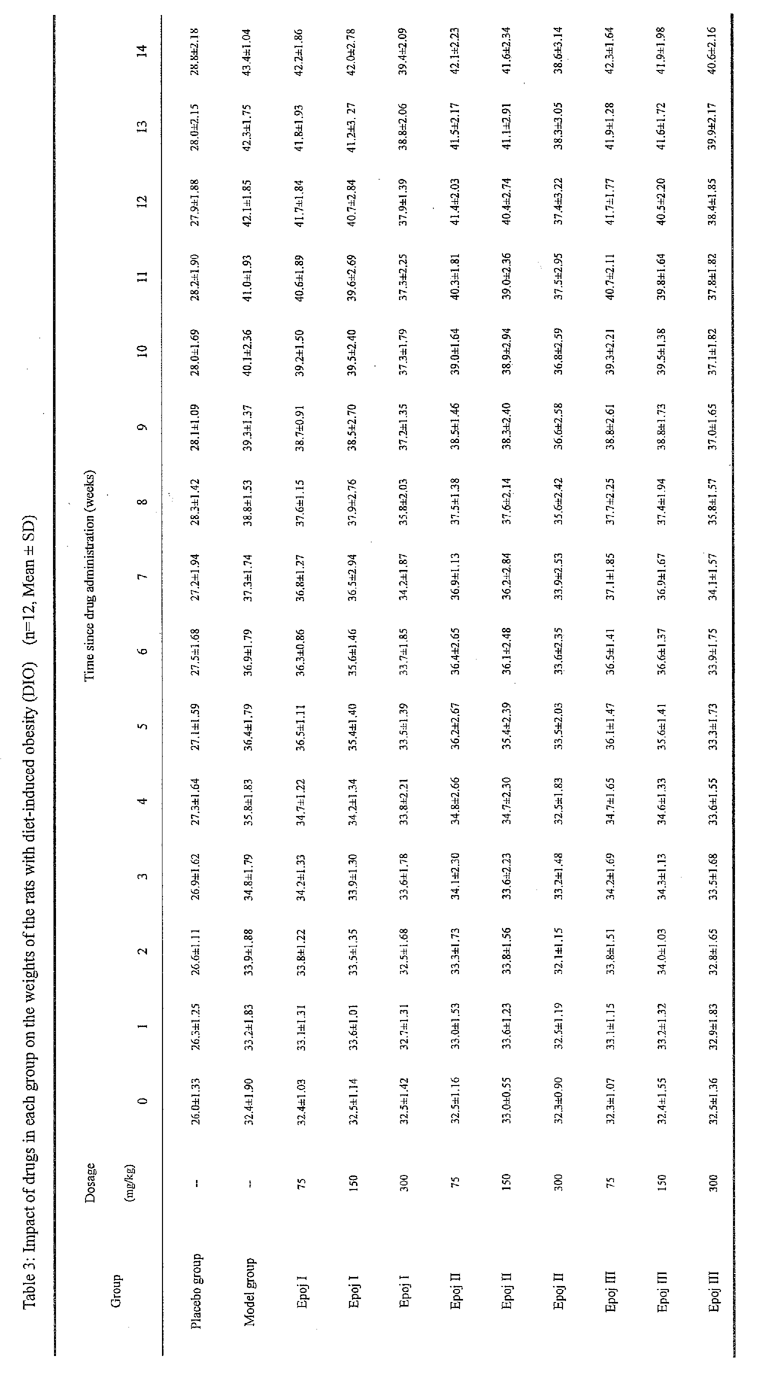

### Industrial Applicability

The present invention employs the model of belly-fat rats to investigate the experiment of the inhibition of the extracts from the polysaccharide of *O. japonicus* on weight gain of the fat rats, to evaluate the weight reducing effects of the extracts from the polysaccharide of *O. japonicus.* The experiment carried out on the belly-fat rats shows that the extracts from the polysaccharide of *O. japonicus* does not affect the food intake quantity of the rats, and that after being administered a high dose of the extracts from the polysaccharide of *O. japonicus* for one week, the weight gain of the rats is substantially mitigated, and the effects of the weight gain inhibition last for 14 weeks till the conclusion of the experiment. Compared with those in the model group, the belly-fat rats administered a high dose of the extracts from the polysaccharide of *O. japonicus* show a substantial decrease in the rate of their weight gain. The outcome of the experiments shows that: the extracts from the polysaccharide of *O. japonicus* of the present invention possesses a prominent weight reducing effect, and is applicable in the preparation of food additives, health products or pharmaceutical products with weight reducing effect.

## Claims

1. A usage of a polysaccharide extracts from Ophiopogon japonicus on preparation of a food additive, a health product or a pharmaceutical product with weight reducing effects, **characterized in that** the polysaccharide extracts from Ophiopogon japonicus has a peak molecular weight in a range of 3000~6500, and is obtained by the following methods:
1) Extracting the polysaccharide of *O. japonicus*
Taking dry tuberous roots of *O. japonicus,* refluxing them three times with 70%-95% ethanol 5-6 times of their weight, discard the roots, extracting a decoction 2-3 times therefrom, concentrating the aqueous decoction under reduced pressure to a concentration of 0.5-1 g/ml, adding 75%-95% ethanol until the ethanol concentration reaches 60%-80%, standing the decoction overnight, discharging the supernatant thereof, carrying out ethanol precipitation twice, drying the decoction, and obtaining a powder of total polysaccharides of *O. japonicus,* which is in pale yellow color;
2) Purifying the extracts from the polysaccharide of *O. japonicus:*
Taking the total polysaccharides of *O. japonicus,* adding in water for dissolution, discharging the sediment therein by centrifugation; into the supernatant therefrom adding 1-2 times its volume 70%-95% ethanol to form sedimentation, standing the supernatant overnight, and discharging the sediment; into the supernatant adding once again 1-2 times its volume 70%-95% ethanol to form sedimentation again, standing the supernatant overnight, and collecting the sediment formed hitherto, which is an extracts from the polysaccharide of *O. japonicus* with a range of molecular weight in 5000~6000; in the remaining supernatant adding in 4-5 times of its volume 70%-95% ethanol to form sedimentation, standing the supernatant overnight, and collecting the remaining sediment, which is an extracts from the polysaccharide of *O. japonicus* with a range of molecular weight in 3000~5000.

2. The usage of Claim 1, **characterized in that** the health product or the pharmaceutical product is an oral formulation.

3. The usage of Claim 2, **characterized in that** the oral formulation comprises 5%-60% by weight of the extracts from the polysaccharide of *O. japonicus,* with the rest components being pharmaceutical excipient or additive.
